# FASCICULE DE BREVET EUROPEEN

(11) **EP 2 282 710 B1**
(45) Date de publication et mention de la délivrance du brevet: **04.04.2012**
(21) Numéro de dépôt: 09757692.0
(22) Date de dépôt: 02.06.2009
(51) Int. Cl.: A61G 7/075, A61G 7/057

(54) **DISPOSITIF DE PROTECTION ANTI ESCARRE DU PIED**
VORRICHTUNG ZUM SCHUTZ DES FUSSES VOR WUNDEN STELLEN
DEVICE FOR PROTECTING THE FOOT AGAINST SORES

(30) Priorité: 04.06.2008 FR 0803091
(43) Date de publication de la demande: 16.02.2011
(73) Titulaire: Roig, Agnès, 34230 Pouzols (FR)
(72) Inventeur: Roig, Agnès, 34230 Pouzols (FR)
(74) Mandataire: Rhein, Alain
(86) Numéro de dépôt international: PCT/FR2009/000632
(87) Numéro de publication internationale: WO 2009/147316

(56) Documents cités:
- DE-C- 853 190
- GB-A- 2 351 024
- US-A- 4 104 746
- US-A- 4 135 504

## Description

### Domaine de l'invention

La présente invention concerne le domaine des appareils et accessoires de soin ou de protection corporelle ou médicale ; et elle intéresse plus particulièrement une enveloppe de protection du talon à des fins curatives ou préventives de l'escarre du talon et/ou des malléoles évitant en outre le syndrome équin et ce dans diverses positions du décubitus;

### Rappel de la technique antérieure

On connaît divers dispositifs destinés au repos et à la protection du pied et de la partie inférieure de la jambe, applicable notamment aux personnes immobilisées en position horizontale pour une période de longue durée. D'une façon générale ces dispositifs visent à éviter des contacts prolongés des parties du pied en saillie contacts prolongés qui provoquent la formation d'escarres ; la présence et la persistance de ces escarres entraine des couts de soins élevés et constitue un problème majeur dans le cadre des budgets affectés à la santé publique.

On connaît ainsi des talonnières dites enveloppantes telles en peau de mouton, à microfibres à garniture gélifiée ou microbilles, permettant de réduire le point de pression ; mais ces accessoires sont peu confortables car nécessitant la mise en place de sangles de maintien et n'assurent pas la position stable du pied et en outre ne protègent pas de l'équinisme.

Des talonnières du type gouttièr voir par exemple DE 853190) ne permettant que le décubitus dorsal et nécessitent l'immobilité stricte des membres inférieurs; et de plus n'étant pas solidaires du pied elles peuvent se révéler instables sous le pied. Des talonnières plates ne s'adaptent pas à toutes les positions et n'évitent pas l'équinisme.

D'une façon générale ces talonnières de type connu si elles ne sont pas exactement positionnées et maintenues, ce qui pose des problèmes pour des patients agités, peuvent engendrer des effets secondaires, tels que l'apparition d'escarres en dehors de la zone protégée, des blessures, contusions ou cisaillements.

### Objet de l'invention

L'invention vise à remédier à ces inconvénients et concerne une enveloppe de protection du pied suivant la revendication 1, visant à dégager et à protéger des pressions génératrices d'escarres les zones à risques telles 1 a face arrière du talon, les malléoles, la base du gros orteil, et ce dans les diverses positions du sujet; en outre le dispositif selon invention protège le pied immobilisé contre le risque d'équinisme.; enfin il permet une adaptation indiférenciée au pied droit comme au pied gauche.

### Caractéristiques de invention

A cet effet l'invention concerne une enveloppe formant coussinet de repos et de protection corporelle, à effet anti escarre, conformé pour s'adapter notamment à la zone talonnière, caractérisé en ce qu'il est constitué d'un bloc en matériau élastiquement déformable et en forme générale de couronne et notamment de tore à gorge , traversé par une pluralité de canaux d'axe radial et ces canaux étant situés dans le plan équatorial du volume torique.

En outre les dites les canaux sont de préférence au nombre de trois et sont disposés selon des méridiens situés sensiblement à équidistance les unes des autres, les dits axes formant entre eux un angle d'environ 120°.

Plus spécialement le coussinet comporte un premier canal de section sensiblement circulaire, propre à envelopper la base de la jambe au dessus des chevilles, un second canal de section sensiblement rectangulaire, propre à envelopper le coup de pied et un troisième canal conformé selon deux troncs de cône opposés par leur sommet, le pourtour du tronc de cône intérieur débouchant dans la gorge centrale étant propre à entourer la zone talonnière.

Et plus spécialement le pourtour du débouché des dits canaux dans la gorge centrale épouse un profil en biseau adouci et curviforme.

Selon une autre caractéristique, le coussinet comporte une fente disposée dans le plan équatorial et courant sur un segment du volume torique compris entre le premier et le second canal, permettant l'ouverture de ce segment en deux parties égales en vue de l'insertion de la zone talonnière à l'intérieur du coussinet, le talon venant en face du troisième canal, le premier canal étant apte à se refermer autour de la base de la jambe, et le coup de pied étant positionné dans le dit second canal.

Selon encore un développement de l'invention, la paroi intérieure de la gorge est prévue avec une rainure annulaire de section curviforme disposée dans le plan équatorial, propre à offrir de chaque coté de la dite gorge un surfaçage adouci et un contact tangentiel de la dite paroi sur chacune des excroissances malléoliennes.

Les deux demi segments du volume torique séparés par la dite fente sont prévus avec des moyens de fermeture et de maintien en contact solidaire des deux moitiés après insertion du pied dans son coussinet.

Et les dits moyens de fermeture et de maintien sont constitués de sangles passant par des lumières disposées en regard sur les deux dites moitiés du segment fendu, les sangles venant de chaque coté du coussinet étant aptes à se réunir et étant prévues à cet effet avec des moyens de solidarisation rapide tels que connus sous la marque commerciale Velcro.

De plus et selon encore une caractéristique de l'invention, le coussinet comporte sur la paroi extérieure du volume torique, située entre le premier et le troisième canal , un méplat propre à permettre un repos stable du coussinet contenant le pied prisonnier, le coussinet reposant par ce méplat sur un plan d'appui, tell que couche ou lit et de préférence ce méplat est disposé à proximité du débouché du troisième canal vers l'extérieur.

Et plus particulièrement le coussinet est prévu avec deux méplats symétriques par rapport au plan équatorial et formant avec le dit plan un angle inférieur à 90° et de préférence compris entre 70 et 80 °.

D'autres caractéristiques et avantages de invention ressortiront de la description donnée à titre d'exemple non limitatif et en relation avec les dessins annexés, dans lesquels :

### Présentation des dessins

La figure 1 représente un pied gauche équipé du coussinet selon l'invention, vu de profil.

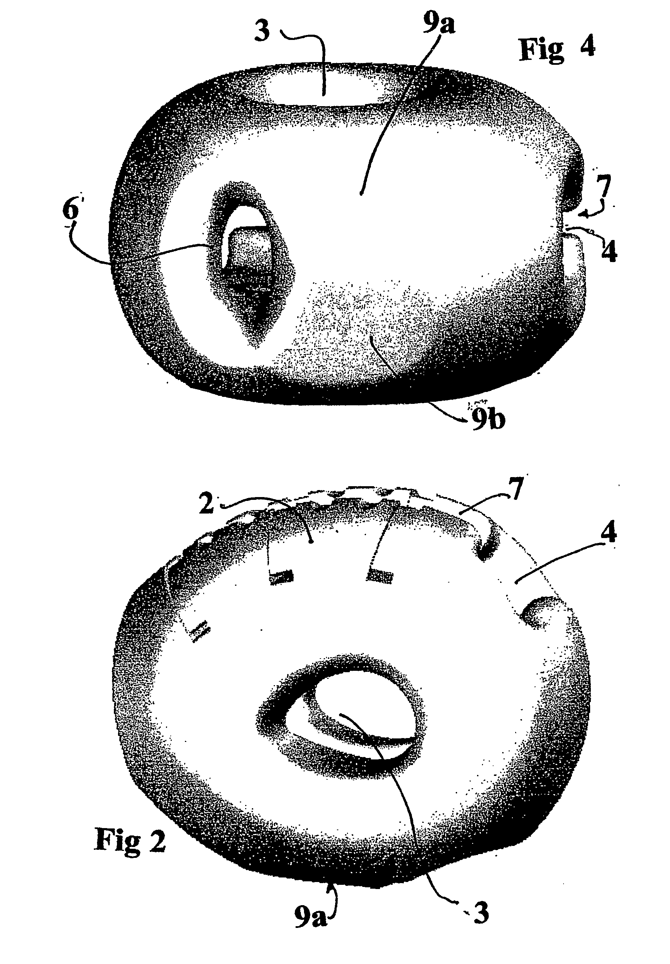

La figure 2 représente le coussinet seul, vue de profil et légèrement de trois quart.
La figure 3 représente une coupe transversale du coussinet selon le plan équatorial du volume dans le but de mettre en évidence les formes à l'intérieur du dispositif.
La figure 4 représente la partie postérieure du coussinet correspopndant à sa face de repos sur un plan d'appui ( couche, literie).
La figure 5 représente la partie supérieure du coussinet correspondant à sa face restant à l'air libre.

### Description d'un exemple de réalisation de l'invention

De forme générale ovoïde, le coussinet est constitué d'un bloc de mousse viscoélastique 1, avec peau de surface imperméable, lavable et désinfectable.

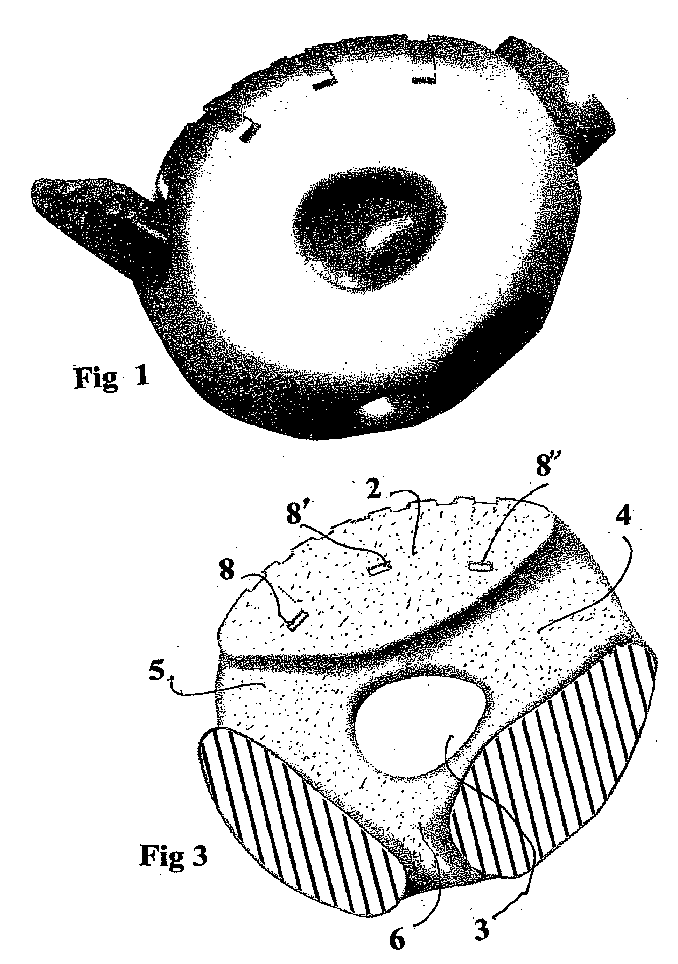

Le bloc forme une couronne torique2 traversée par une gorge centrale3 disposée selon un axe orthogonal au plan équatorial du volume torique, plan correspondant au plan de coupe de la figure 3.

Trois canaux (visibles notamment sur la vue en coupe de la figure 3) traversent la couronne, partant de sa paroi extérieure et débouchant dans l'intérieur de la gorge centrale.3 Soit un premier canal 4 de section sensiblement circulaire, destiné à emboiter la base de la jambe au dessus de la cheville, un second canal 5 de coupe sensiblement rectangulaire est prévu pour emboiter le coup de pied; un troisième canal 6 épouse la configuration de deux troncs de cône opposés par leur sommet, les bases des dits cônes débouchant d'une part sur la paroi extérieure de la couronne torique et de l'autre coté vers l'intérieur de la gorge.

Les axes de ces canaux radiaux suivent ensemble une forme générale de Y et occupent des méridiens du volume torique sensiblement équidistants, Le troisième canal biconique restant dégagé pour l'aération et le dégagement du talon forme ainsi le montant central du dit Y, les premier et second canaux récepteurs du pied forment les deux branches de ce Y, en formant entre eux un angle supérieur à 120 °, compris par exemple entre 130 et 140 °..

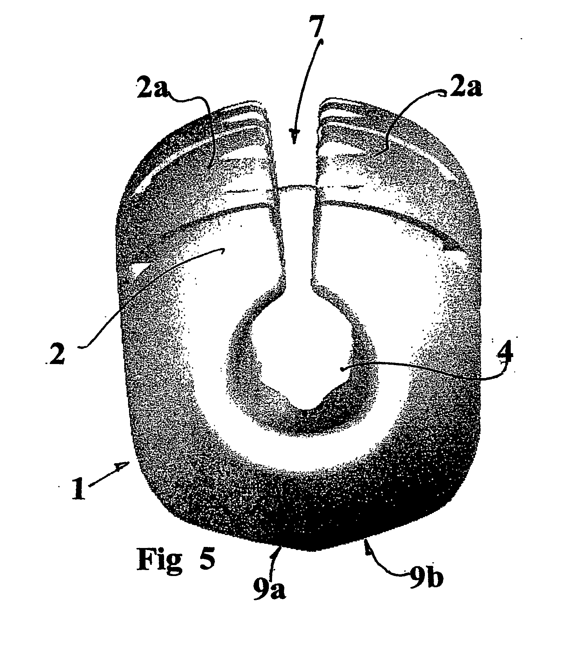

Une fente 7 (bien visible sur, la figure 5) suivant le plan équatorial du volume torique coupe en deux parties égales 2a le segment 2 de la couronne torique compris entre le premier et le second canal , respectivement 4 et 5 ; elle permet l'ouverture du volume pour le passage du pied venant occuper les dits premier 4 et second 5 canaux. ; ces deux demi segments sont traversés par des lumières 8, 8', 8" en concordance d'une moitié à l'autre pour recevoir des sangles (non représentées) permettant de resserrer les deux demi segments l'un contre l'autre autour du creux supérieur du pied après son insertion et sa .mise en place dans les deux premier 4 et second 5 canaux.

Ces sangles (analogues à des lacets de souliers) permettent ainsi de resserrer la fente 7 et d'assurer le rapprochement et le maintien des deux demi segments latéraux dans des position proches, serrées autour de la base de la jambe ;
On assure ainsi le confort de l'usager avec l'immobilisation de la protection autour du pied sujet à contention et/ou protection, et ce malgré les mouvements volontaires ou spontanés qui peuvent affecter la jambe et le pied concerné.

La gorge centrale 3 du volume torique de forme générale ovoïde permet de recevoir l'appui du pourtour des malléoles internes et externes dont les excroissances se trouvent ainsi dégagées et libres de tout frottement par rapport à la surface de repos du patient ; les bords internes de la gorge 3 présentent une rainure équatoriale curviforme concave évitant toute arête ou forme en saillie dans la zone d'appui sur le pourtour des malléoles;
Le creux interne de la gorge forme ainsi deux cônes opposés par leur base, chaque cône en creux épousant la forme générale conique en relief de chacune des malléoles, le dispositif de l'invention formant ainsi pour chacune ces malléoles un berceau récepteur de forme complémentaire des reliefs protégés.

Deux méplats 9a et 9b symétriques par rapport au plan équatorial sont destinés à permettre un appui du coussinet sur sa surface de repos, pour tenir compte de la décline naturelle du pied au repos, et un soutien suffisant au niveau du talon d'Achille ; il est compris que chacun de ces méplats de repos du coussinet est d'utilisation alternative, un seul méplat étant en position active d'appui ; en assurant une légère inclinaison vers l'extérieur du plan équatorial du coussinet par rapport a un plan vertical idéal ; Un méplat correspond ainsi à une utilisation du coussinet sur pied droit et l'autre méplat à utilisation du coussinet sur pied gauche.

Le coussinet de l'invention sera avantageusement fabriqué à partir d'un moule dans lequel est injectée une résine thermoplastique à température convenable émulsifiable en vue d'obtenir un bloc en mousse visco-élastique.à cellules ouvertes.

Ce type de fabrication, par injection dans un moule suivie ou accompagné d'une émulsification , avec formation d'une peau superficielle, relève de techniques connues dans le domaine de la plasturgie. Le choix de la résine n'est pas caractéristique , dés lors que la mousse obtenue répond aux caractéristiques physiques de visco-élasticité exposées dans le cahier des charges des supports d'aire à la prévention de l'escarre.. Néanmoins les essais et expérimentations auxquelles il a été procédé sous la direction de l'inventeur ont conduit a préconiser l'utilisation d'une mousse à base de polyuréthane.

De façon connue les réactifs (polyol et diisocyanate), sont mélangés immédiatement avant leur injection dans un moule ; le mélange comportant les adjuvants conventionnels, tels que plastifiant, agents d'émulsification, devant respecter les exigences de contact avec la peau. humaine; la formation d'une couche superficielle ou "peau" sur l'objet lui-même est obtenue par des opérations thermiques connues et assurera l'imperméabilisation de l'objet et par conséquent des facilités de nettoyage et désinfection.

Le dosage des réactifs, adjuvants et le cycle opératoire ( durée, température) sera calculé et ajusté selon les techniques du plasturgiste de façon à obtenir une mousse de résilience appropriée et de préférence du type haute résilience.
Un élément important dans les paramètres a déterminer dans le processus de fabrication est la densité ou masse volumique. Ici encore les essais et expérimentations auxquels le coussin talonnier, objet de l'invention, a été soumis, conduisent à retenir une valeur préférentielle, telle que ci- après exposée.

On rappelle préalablement que dans les produits répondant aux mêmes fins, connus dans l'art antérieur, il est fait usage de mousses présentant des masses volumiques de l'ordre de 60 à 80 kg/ mètres cube, jugée nécessaire pour atteindre les conditions d'un suportage stable. Ce qui conduit a des modules unitaires présentant un poids (pour chaque membre inférieur) de l'ordre de 600 grammes; ce poids porté en permanence par les pieds du sujet se révèle handicapant.

La forme nouvelle et spécifique du coussin selon la présente invention, permettant une parfaite adaptation à la morphologie du membre protégé; .tout en assurant un repos stable mais non contraignant, permettent d'utiliser et de recommander l'emploi d'une mousse plus légère et de densité ou masse volumique comprise entre 40 et 60 kg / mètre cube, et de préférence restant voisine de 45 kg/m3 (pour un pied, moyen).
De ce fait on obtient un coussin unitaire dont le poids peut se situer au niveau de 300 g, donc un poids réduit de moitié par rapport aux dispositifs antérieurs , ce qui améliore considérablement le confort et la liberté de mouvement du sujet.

Les dimensions de la protection de l'invention sont déterminées par la morphologie des sujets de sorte que l'on pourra prévoir divers modèles échelonnés de façon a répondre aux différentes tailles de pieds à traiter ou protéger.
A titre d'exemple pour un pied considéré comme moyen, dit chaussant du 39/40 , le diamètre hors tout du bloc torique pourra être de l'ordre de 30 à 35 cm , avec gorge centrale de diamètre voisin de 10 à 12 cm.

Le coussinet de l'invention est destiné principalement aux sujets à mobilité faible ou nulle, notamment les personnes âgées, les handicapés moteurs, malades en soins intensif, soins palliatifs.

Grâce à l'élimination de la pression au niveau du talon et des malléoles dans toutes les positions du décubitus, le dispositif selon la présente invention permet l'éradication de l'escarre talonnière et malléolienne (50% des escarres totales) tout en protégeant de l'équinisme. Il pourra être utilisé en prévention mais aussi de manière curative, associé par exemple à un pansement.

La description qui précède n'a été donnée qu'à titre exemplatif en relation avec une forme particulière de réalisation et on pourra adopter diverses variantes ou adaptations sans sortir des limites de l'invention. La description donnée porte sur la version de l'invention retenant une forme torique à gorge du coussinet; mais la morphologie torique n'est pas rigoureusement nécessaire et a été retenue pour la souplesse et l'aisance aux mouvements qu'elle procure au sujet; une version du coussinet de l'invention adoptant la forme d'une couronne, ou s'agissant d'un volume d'une section de cylindre tubulaire à base constituée d'une couronne pourra convenir.

De plus, l'invention a été décrite en relation avec son application à une protection anti escarre du pied, application qui n'est pas limitative; le coussinet de l'invention peut être utilisé dans tous les cas, notamment de blessure, plaie ou fracture, déformation osseuse, ou encore affection de nature dermatologique, où l'on souhaite éviter un contact de repos ou d'appui du pied ou lorsque le pied doit être maintenu inactif et protégé de contacts.

## Revendications

1. Coussinet de repos et de protection corporelle, notamment à effet anti escarre, conformé pour s'adapter notamment à la zone talonnière, **caractérisé en ce qu'**il est constitué d'un bloc en matériau élastiquement déformable (1) et en forme générale de couronne et spécialement de tore à gorge (3), traversé par une pluralité de canaux (4, 5 et 6) d'axes radiaux et situés dans le plan équatorial du volume.

2. Coussinet anti escarre selon la revendication 1, **caractérisé en outre en ce que** les canaux, (4, 5 et 6) sont au nombre de trois, chacun s'ouvrant d'un coté sur la paroi extérieure du volume notamment torique et débouchant de l'autre coté sur l'intérieur de la gorge centrale, et sont disposés selon des méridiens situés sensiblement à équidistance les unes des autres, les dits axes formant entre eux un angle voisin de 120°.

3. Coussinet anti escarre selon la revendication 2, **caractérisé en ce qu'**il comporte un premier canal (4) de section sensiblement circulaire, propre à envelopper la base de la jambe au dessus des chevilles, un second canal (5) de section sensiblement rectangulaire, propre à envelopper le coup de pied et un troisième canal (6) conformé de préférence selon deux troncs de cône opposés par leur sommet, le pourtour du tronc de cône intérieur débouchant dans la gorge centrale (3) étant propre à entourer la zone talonnière.

4. Coussinet anti escarre selon l'une des revendications 1 à 3 ci-dessus, **caractérisé en ce que** le pourtour du débouché des canaux dans la gorge centrale épouse un contour en biseau adouci et curviforme.

5. Coussinet selon l'une des revendications 1 à 4 ci-dessus, **caractérisé en ce qu'**il comporte une fente (7) disposée dans le plan équatorial de la couronne torique et courant sur un segment (2a) du dit volume torique compris entre le premier (4) et le second (5) canal, permettant l'ouverture de ce segment en deux parties égales en vue de l'insertion de la zone talonnière à l'intérieur du coussinet, le talon venant en face du troisième canal (6), le premier canal (4) étant apte à se refermer autour de la base de la jambe, et le coup de pied étant positionné dans le dit second canal (5).

6. Coussinet selon la revendication 5, **caractérisé en ce que** la paroi intérieure de la gorge (3) est prévue avec une rainure annulaire de section en creux curviforme ainsi propre à offrir sur la paroi de la dite gorge un surfaçage circulaire en creux adouci et un contact tangentiel de la dite paroi sur chacune des excroissances malléoliennes.

7. Coussinet selon la revendication 5, **caractérisé en ce que** les deux moitiés du segment (2a), formant chacune un demi segment du volume notamment torique séparé par la dite fente (7) sont prévus avec des moyens de resserrement de la dite fente et de maintien dans des positions mutuellement proches des deux moitiés ou demi segments après insertion du pied dans son coussinet.

8. Coussinet selon la revendication 7 et **caractérisé en ce que** les dits moyens de fermeture et de maintien sont constitués de sangles passant par des lumières (8, 8', 8") disposées en regard sur les deux dites moitiés du segment (2a) fendu, les sangles venant de chaque coté du coussinet étant aptes à se réunir sur la paroi extérieure du segment (2a) et ces sangles étant prévues à cet effet avec des moyens de solidarisation rapide tels que connus sous la marque commerciale Velcro.

9. Coussinet selon l'une des revendications 1 à 8 ci-dessus, **caractérisé en ce qu'**il comporte sur la paroi extérieure du volume notamment torique, située entre le premier (4) et le troisième (5) canal au moins un méplat 9a propre à permettre un repos stable du pied prisonnier dans son coussinet et reposant sur un plan d'appui,
tel que couche ou lit et de préférence ce méplat est disposé à proximité du débouché du troisième canal (6) vers l'extérieur,
et le coussinet comporte de préférence deux méplats symétriques (9a et 9b) par rapport au plan équatorial et formant avec le dit plan un angle inférieur à 90° et de préférence compris entre 70 et 80°.

10. Coussinet selon l'une des revendications 1 à 9, **caractérisé en ce qu'**il est constitué d'une mousse visco-élastique et de préférence de mousse de polyuréthane à cellules ouvertes, pourvu d'un peau superficielle étanche et de masse volumique comprise entre 40 et 60 kg/ mètre cube, et de préférence encore voisine de 45 kg/m3.

## Claims

1. Resting and body protecting cushion, in particular with an anti-scab effect, designed so as to be adapted to the heel area, wherein it is formed of a block made of elastically deformable material (1) and having generally the shape of a crown and in particular of a grooved torus (3), through which pass a plurality of channels with a radial axis (4, 5 and 6) located in the equatorial plane of the volume.

2. Anti-scab cushion according to claim 1, wherein furthermore the channels (4, 5 and 6) are three in number, each one opening on one side on the outer wall of the namely annular volume, and ending on the other side at the interior of the central groove, and are arranged according to meridians located substantially equidistant from each other, said axes forming between them an angle close to 120°.

3. Anti-scab cushion according to claim 2, wherein that it comprises a first channel (4) having a substantially circular cross-section, suitable for wrapping the leg base above the ankles, a second channel (5) having a substantially rectangular cross-section, suitable for wrapping the ankle, and a third channel (6) preferably designed according to two cone stems opposed by their tops, the circumference of the inner cone stem ending into the central groove (3) suitable for wrapping the heel area.

4. Anti-scab cushion according to one of the above-mentioned claims 1 to 3, wherein the circumference of the ending of the channels in the central groove matches a slightly beveled and curved contour.

5. Anti-scab cushion according to one of the the above-mentioned claims 1 to 4, wherein it comprises a slit (7) arranged in the equatorial plane or the annular crown and running over a section (2a) of said annular volume comprised between the first (4) and the second (5) channel, allowing the opening of said section into two equal parts in order to insert the heel area into the cushion, the heel facing the third channel (6), the first channel (4) being capable of closing around the leg base, and the ankle being positioned in said second channel (5).

6. Cushion according to claim 5, wherein the interior wall of the groove (3) is designed with an annular groove having a curved hollow cross-section, thus suitable to provide on the wall of said throat a slightly hollowed circular surfacing and a tangential contact with said wall on each of the malleolus excrescences.

7. Cushion according to claim 5, wherein the two halves of the section (2a), each forming a half-section of the namely annular volume separated by said slit (7), are provided with means for tightening said slit and maintaining in mutually close positions the two halves or half-sections after insertion of the leg in its cushion.

8. Cushion according to claim 7 and wherein said closing and maintaining means are formed by straps passing through holes (8, 8', 8") arranged opposite each other on the two halves of the split section (2a), the straps extending on both sides of the cushion being capable of being united on the outer wall of the section (2a) and these straps being provided to this end with quick fastening means, such as known under the Velcro trade mark.

9. Cushion according to one of the above-mentioned claims 1 to 8, wherein it comprises on the outer wall of the namely annular volume, located between the first (4) and the third (6) channel at least one flat 9a suitable for allowing a stable resting of the foot inserted into the cushion, and resting on a supporting plane, such as a layer or bed, and this flat is preferably arranged proximate of the ending of the third channel (6) towards the exterior,
and the cushion preferably comprises two flats (9a and 9b) symmetrical with respect to the equatorial plane and forming with said plane an angle smaller than 90° and preferably varying between 70° and 80°.

10. Cushion according to one of claims 1 to 9, wherein it is formed of viscoelastic foam and preferably of open-cell polyurethane foam, provided with a waterproof surface skin with a mass density comprised between 40 and 60 km/cubic meter, and preferably clsose to 45 kg/m3.

## Patentansprüche

1. Fersenschoner zur Druckentlastung und zum Körperschutz, insbesondere mit Antidekubituswirkung, der angepasst ist, um sich insbesondere an den Fersenbereich anzupassen, **dadurch gekennzeichnet, dass** er durch ein Ganzstück aus einem elastisch verformbaren Werkstoff (1) und mit der allgemeinen Form eines Kranzes und insbesondere eines Torus mit einem Mittelloch (3) gebildet ist, der von einer Mehrheit von Kanälen (4, 5 und 6) mit radialen Achsen durchquert ist, die sich auf der Mittelhöhe des Volumens befinden.

2. Antidekubitus-Fersenschoner nach Anspruch 1, der ferner **dadurch gekennzeichnet ist, dass** die Kanäle (4, 5 und 6) drei an der Zahl sind, die jeweils einerseits in die Außenwand des insbesondere torischen Umfangs münden, und andererseits zum Inneren des Mittellochs führen, und die entlang den Meridianen angeordnet sind, die im Wesentlichen in gleichem Abstand voneinander verlaufen, wobei die besagten Achsen untereinander einen Winkel im Bereich von etwa 120° bilden.

3. Antidekubitus-Fersenschoner nach Anspruch 2, **dadurch gekennzeichnet, dass** er einen ersten Kanal (4) mit einem im Wesentlichen kreisförmigen Querschnitt, geeignet, um den unteren Teil des Unterschenkels oberhalb den Knöcheln zu umschließen, einen zweiten Kanal (5) mit einem im Wesentlichen rechteckigen Querschnitt, geeignet, um den Fußrücken zu umschließen, und einen dritten Kanal (6) umfasst, der vorzugsweise in der Art von zwei Kegelstümpfen ausgestaltet ist, die mit ihren Spitzen einander gegenüberliegen, wobei der Umriss des inneren Kegelstumpfs, der zum Mittelloch (3) führt, geeignet sei, um den Fersenbereich zu umfangen.

4. Antidekubitus-Fersenschoner nach einem der obenstehenden Ansprüche 1 bis 3, **dadurch gekennzeichnet, dass** der Umriss des Einmündung der Kanäle in das Mittelloch sich einem weich abgeschrägten und abgerundeten Konturverlauf anschmiegt.

5. Fersenschoner nach einem der obenstehenden Ansprüche 1 bis 4, **dadurch gekennzeichnet, dass** er einen Spalt (7) umfasst, der sich auf der Mittelebene des torischen Kranzes befindet und einen Teilbereich (2a) des besagten torischen Umfangs, welcher zwischen dem ersten (4) und dem zweiten (5) Kanal umschlossen ist, durchstreift, erlaubend die Auffaltung dieses Teilbereichs in zwei gleiche Hälften zwecks Einsetzen des Fersenbereichs in das Innere des Fersenschoners, wobei die Ferse dem dritten Kanal (6) zugewandt ist, wobei der erste Kanal (4) geeignet sei, um sich um den unteren Teil des Unterschenkels herum zu verschließen, und wobei der Fußrücken in dem besagten zweiten Kanal (5) positioniert sei.

6. Fersenschoner nach Anspruch 5, **dadurch gekennzeichnet, dass** die innere Wand des Mittellochs (3) mit einer ringförmigen Einkerbung mit einem abgerundeten Hohlquerschnitt vorgesehen ist, die so geeignet sei, um in der Wand des besagten Mittellochs eine weiche kreisförmige Hohlfläche und einen tangentialen Kontakt der besagten Wand mit jeder Knöchel-Exkreszenz zu erlauben.

7. Fersenschoner nach Anspruch 5, **dadurch gekennzeichnet, dass** die beiden Hälften des Teilbereichs (2a), die jeweils eine Segmenthälfte des insbesondere torischen, durch den besagten Spalt (7) aufgespaltenen Umfangs bilden, mit Mitteln zur Straffspannung des besagten Spaltes und zum Halten der beiden Hälften oder Segmenthälften in eng zueinander herannahenden Positionen nach dem Einsetzen des Fußes in seinen Fersenschoner vorgesehen sind.

8. Fersenschoner nach Anspruch 7 und **dadurch gekennzeichnet, dass** die besagten Mittel zur Straffspannung und zum Halten durch Schnürriemen gebildet sind, die durch die Öffnungen (8, 8', 8") hindurchgehen, welche an den besagten beiden Hälften des aufgespaltenen Teilbereichs (2a) einander gegenüberliegend angeordnet sind, wobei die Schnürriemen, die von jeder Seite des Fersenschoners kommen, geeignet seien, um sich auf der Außenwand des Teilbereichs (2a) wieder miteinander zu verknüpfen, und diese Schnürriemen zu diesem Zweck mit Schnellverschlussmitteln wie jene, die unter der Handelsmarke Velcro bekannt sind, vorgesehen seien.

9. Fersenschoner nach einem der obenstehenden Ansprüche 1 bis 8, **dadurch gekennzeichnet, dass** er auf der Außenwand des insbesondere torischen Umfangs, die zwischen dem ersten (4) und dem dritten (5) Kanal angeordnet ist, zumindest eine Abflachung 9a umfasst, die geeignet ist, um eine stabile Ruheposition des in seinem Fersenschoner eingeschlossen und auf einer Abstützebene wie eine Auflageschicht oder ein Fußbett ruhenden Fußes zu erlauben, und dadurch, dass diese Abflachung vorzugsweise nahe der Mündung nach Außen des dritten Kanals (6) angeordnet ist, und dadurch, dass der Fersenschoner vorzugsweise zwei Abflachungen (9a und 9b) umfasst, die hinsichtlich der Mittelebene symmetrisch sind und mit der besagten Ebene einen Winkel bilden, der kleiner als 90° ist und vorzugsweise zwischen 70° und 80° liegt.

10. Fersenschoner nach einem der Ansprüche 1 bis 9, **dadurch gekennzeichnet, dass** er aus einem viskoelastischen Schaumstoff und vorzugsweise aus einem offenzelligen Polyurethanschaumstoff besteht, der mit einer dichten Oberflächenhaut versehen ist und eine Dichte aufweist, die zwischen 40 und 60 kg/Kubikmeter und insbesondere bevorzugt im Bereich von etwa 45 kg/m3 liegt.
